# EUROPEAN PATENT APPLICATION

(11) **EP 1 269 946 A1**
(43) Date of publication of application: **02.01.2003**
(21) Application number: 01114145.4
(22) Date of filing: 11.06.2001
(51) Int. Cl.: A61F 5/451

(54) **Human waste collection bag of improved shape**

(71) Applicant: THE PROCTER & GAMBLE COMPANY, Cincinnati, Ohio 45202 (US)
(72) Inventor: Sugita, Masataka, Osaka (JP); Hasegawa, Maya, Ashiya City (JP); Burns, Jay G., Kobe City (JP)
(74) Representative: Hirsch, Uwe Thomas M.H.

(57) **Abstract**

The present invention relates to human waste collection bags for babies, children or adults to be attached to a wearer. More particularly it relates to a human waste collection bag comprising a pouch (11), the pouch (11) comprising a wearer facing portion (16) and a garment facing portion (17), both having a surface area, characterised in that the surface area of the garment facing portion (17) is greater than the surface area of the wearer facing portion (16). In another aspect the present invention relates to a pouch (11) which comprises a material having a stiffness of less than 20 according to the test described herein.

## Description

### Field of the invention

The present invention relates to human waste collection bags for babies, children or adults to be attached to a wearer. More particularly it relates to the shape of the pouch comprised by such a collection bag.

### Background of the invention

Human waste collection bags are known articles of manufacture that are designed to be worn principally by incontinence sufferers and in particular by bedridden patients. Such human waste collection bags are attached to the perianal area or uro-genital area of the wearer and are intended to entrap and immediately contain faecal material, urine and other bodily discharges.

Faecal collection bags as they are mostly known today are constituted of a relatively long and narrow tube, at one extremity of which is positioned the aperture and the attachment device, which can be adhesive.

Because of their shape and dimensions, such devices can twist around the thighs of the wearers and lead to folds and kinks in the devices themselves. Such features naturally affect the storage capacity of the device and may result in unintentional detachment of the device from the wearer leading to undesirable and distressing consequences both for the wearer and carer. Moreover such devices hinder the free movement of the wearer.

### Such bags are disclosed in e. g. the following documents:

US 3,577,989, which details a disposable elimination-trapping bag for incontinence sufferers including a container member having an open-top portion, and a flange secured to the container member around the open-top portion. The container member includes two opposed side members, preferably substantially identical, and of a generally rectangular configuration, joined together along common edges. US 4,784,656, which describes a receptacle for collecting faecal matter from incontinence sufferers. The faecal collector comprises a gasket, conduit means or a cylinder and a receptacle; the receptacle and conduit means are each formed from two sheets of thermoplastic film that are heat sealed along their side edges, respectively. GB 2 152 387, which teaches a faecal collector for incontinence sufferers comprising a collection bag and a ring. The faecal collector comprises a pair of panels of thermoplastic sheet material joined at their margins to define an elongate bag having an opening at one end. In a preferred embodiment, the collection bag is formed from a single sheet of odour-barrier thermoplastic film folded along a vertical midline to provide a pair of continuous panels. SE 8 104 934, which discloses an oblong bag made from a thin, flexible and fluid tight material. The collecting bag comprises an inlet portion and a bottom portion at an angle of 120 degrees to the longitudinal direction of the inlet portion. The bag is so designed as to enable it to assume an advantageous position along the thigh of the person when in use.

Other types of faecal management bags of a flatter shape are known from EP 245 064. Such types of flatter bags are also disclosed in US 4,946,720, particularly suited however for collecting faecal matters discharged through the artificial anus.

EP 245 064 discloses bags having a front and a rear wall, the front wall containing the aperture and attachment means to the body. The general shape of said front and rear wall is rectangular, i. e. the bag has two opposed long sides and two opposed short sides, the width of the bags being relatively short compared to the length of the bag. Furthermore, the aperture in the front wall is positioned close to one of said short sides of said front wall. Such bags, while improved over the tube-type bags described hereinabove, are still not exhibiting optimum containment properties, especially not in a wide range of wearing conditions.

The wearing conditions will depend on the nature of the wearer; when the wearer is active, such as a baby or a child, or an incontinent adult not being bedridden, the wearing conditions for the bag will become much more stressed and the risk of detachment of the bag will increase substantially, due to the movement of the wearer and pressure from the wearer's body, if the containment properties are not optimum, i. e. there is a likelihood that the faecal material, once excreted and contained in the bag, will exert pressure, in particular onto the inner periphery of the flange, which may result in the unintentional detachment of the bag. Sitting on the bag, for example, will result in a largely reduced volume in some areas of the bag.

Furthermore such pressure exerted by the entrapped faecal material occurring in various wearing conditions may lead to the rupture of the bag, which is an undesirable and distressing consequence, even if only minor leakage of the bag is induced by a rupture. The seals, where different pieces of material used for the bag are joined, e.g. by heat sealing, are typically less resistant to rupture under pressure than other areas of the bag. The weakest areas along such seals are typically bends occurring at the corners of a bag, e.g. when made of essentially rectangular pieces of material as described in the prior art. Bags for faecal management devices are typically made in mass production under economic constrains. Thus a bag having a shape which avoids the need for particularly high quality seals is of high economic benefit for the producer and also provides an at least psychological assurance for the user.

It has now been found that the above drawbacks will be substantially alleviated if the bags are configured in a specific manner, thus allowing utilisation of the bags for babies, children and active adult incontinents in addition to bedridden adult incontinents.

### Summary of the invention

The present invention relates to human waste collection bags for babies, children or adults to be attached to a wearer. More particularly it relates to a human waste collection bag comprising a pouch (11), the pouch (11) comprising a wearer facing portion (16) and a garment facing portion (17), both having a surface area, characterised in that the surface area of the garment facing portion (17) is greater than the surface area of the wearer facing portion (16). In another aspect the present invention relates to a pouch (11) which comprises a material having a stiffness of less than 20 according to the test described herein.

### Brief description of the drawings

It is believed that the invention will be better understood from the foregoing description in conjunction with the accompanying drawings in which:
Figure 1 is a perspective view of a preferred embodiment of a faecal collection bag, the wearer facing portion and the garment facing portion being shown as separate parts.
Figure 2 is an on-top view of a preferred embodiment of a faecal collection bag according to the present invention.
Figure 3 is a rear view of the embodiment of Figure 2.
Figure 4 is a cross-sectional view of the embodiment of Figure 2, the crosssection being made along a longitudinal plane.
Figure 5 is a cross-sectional view of the embodiment of Figure 2, the cross-section being made along a transversal plane.

### Detailed description of the invention

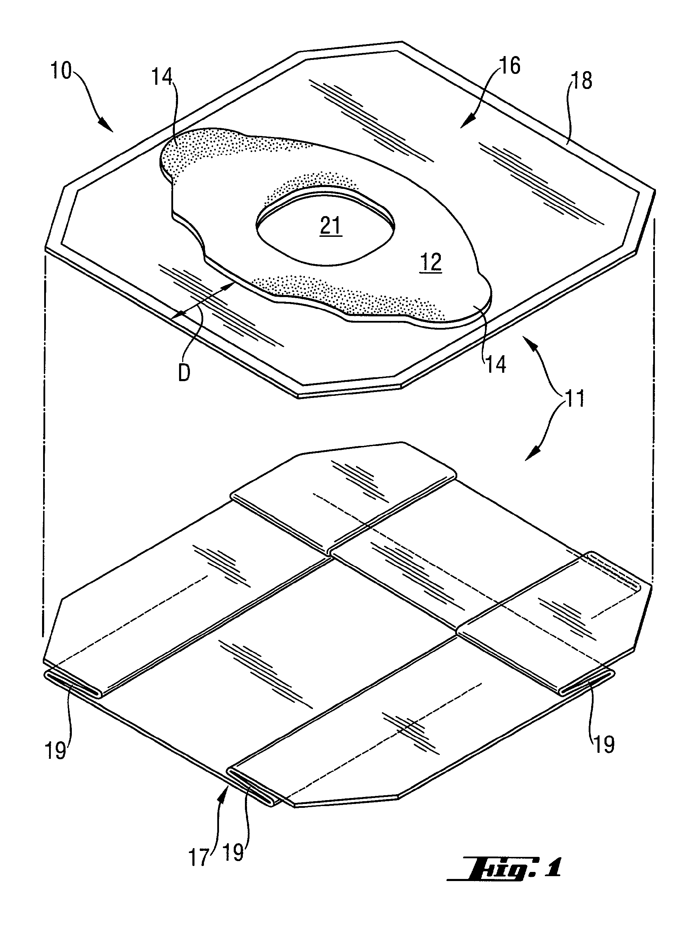

Human waste collection bags comprise faecal collection bags (10) and urine collection bags. Both types of collection bags can comprise similar or alike components and materials and are therefore described together. Figure 1 shows a faecal collection bag (10) according the present invention. Faecal collection bags (10) are designed for attachment to the anal area and mainly used for collecting faeces, whereas urine collection bags are attached to the urinary duct and mainly used for collecting urine. The present invention is preferably applied to faecal collection bags (10) and urine collection bags, but is also applicable to ostomy devices. All of the above human waste collection bags are preferably designed for single use and disposal thereafter.

Typically human waste collection bags comprise a pouch (11) having an aperture (21) and a flange (12) surrounding the aperture for preferably adhesive attachment to the perianal area of a wearer as visible from Figure 1. Any human waste collection bag known in the art can be provided according to the present invention.

The pouch (11) as used herein is a flexible receptacle for the containment of excreted faecal matter or urine. The pouch (11) is designed to safely contain any entrapped material, typically it will be liquid impermeable, yet it may be breathable. The pouch (11) is designed of sufficient strength to withstand rupture in use, also when pressure on the pouch (11) is exerted in typical wearing conditions, such as sitting.

According to the present invention the pouch (11) is provided in a specific shape and comprises a specific material as to improve the containment properties of the pouch, to avoid detachment and to be able to use a high portion of the volume of the pouch for actual storage of faecal matter or urine.

The pouch will comprise a wearer facing portion (16) and a garment facing portion (17). As used herein the wearer facing portion (16) is to be understood as the portion of the pouch (11) generally facing towards the wearer and the garment facing portion (17) is to be understood as the portion of the pouch (11) generally facing the garment of the wearer. When a diaper is worn in addition to a human waste collection bag the garment facing portion (17) will be in contact with the diaper.

Depending on the shape of the pouch (11) required, the pouch (11) may be provided from a unitary piece of material or a number of separate pieces of material, which may be identical or different and which are sealed at their respective peripheries. The preferred shape of the pouch (11) depends in particular on the intended use thereof, i.e. whether the device is intended for bedridden patients or active patients suffering from incontinence or requiring an artificial bowel or for infants.

The pouch (11) described herein preferably have a wearer facing portion (16) and a garment facing portion (17), which both comprise separate pieces of material. The wearer facing portion (16) and the garment facing portion (17) are sealed at the periphery of the pouch (11), thus creating a bag peripheral rim (18). The wearer facing portion (16) and the garment facing portion (17) may each independently comprise more than one section of material. Preferably the garment facing portion (17) comprises only one section of material; most preferably also the wearer facing portion (16) comprises only one section of material.

The wearer facing portion (16), the garment facing portion (17) and the pieces of material comprised by either of these portions are secured to each other by means known to the man skilled in the art, such as adhesive, thermobonding or pressure bonding in order to provide the desired bag configuration. The rim (18), at which the wearer facing portion (16) and the garment facing portion (17) are sealed together, may be provided inside the pouch (11) rather than outside the pouch (11), thus being coextensive with the inner surface of the pouch (11) rather than with the outer surface of the pouch (11).

According to the present invention the shape of the pouch should be chosen such that the surface area of the garment facing portion (17) is greater than the surface area of the wearer facing portion (16). Preferably the surface area of the garment facing portion (17) is at least 10%, preferably 25%, yet preferably 50%, yet more preferably 75% greater than the surface area of the wearer facing portion (16). When the garment facing portion (17) comprises folds (19) as in one preferred embodiment of the present invention the surface area of the garment facing portion (17) is to be understood as the surface area when the garment facing portion (17) is unfolded, ie. corresponds to the surface area of the sheet of material used to provide the garment facing portion (17).

For purposes of packaging and to increase the wearing comfort of the device when no or only few faecal matter or urine are stored, the garment facing portion (17) should preferably be provided in a folded condition. While many folding patterns are suitable, preferred patterns will allow ease of manufacture and ready unfolding when pressure from within the bag is acting upon the folds (19).

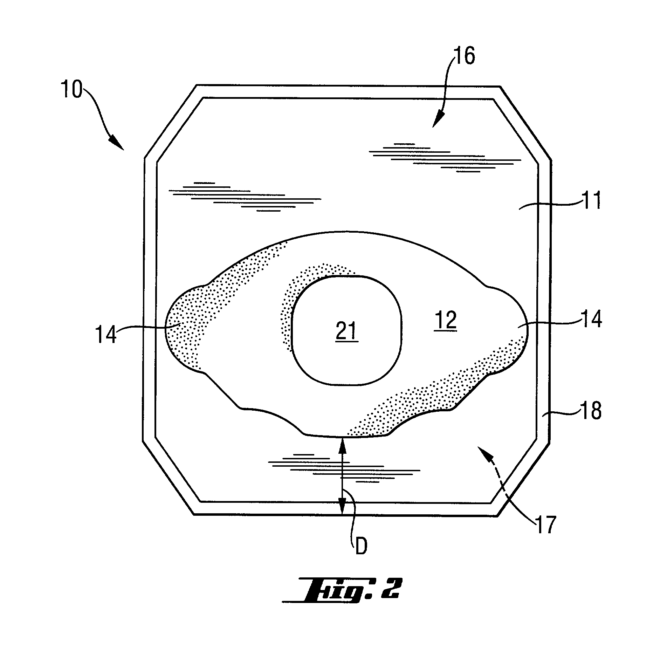

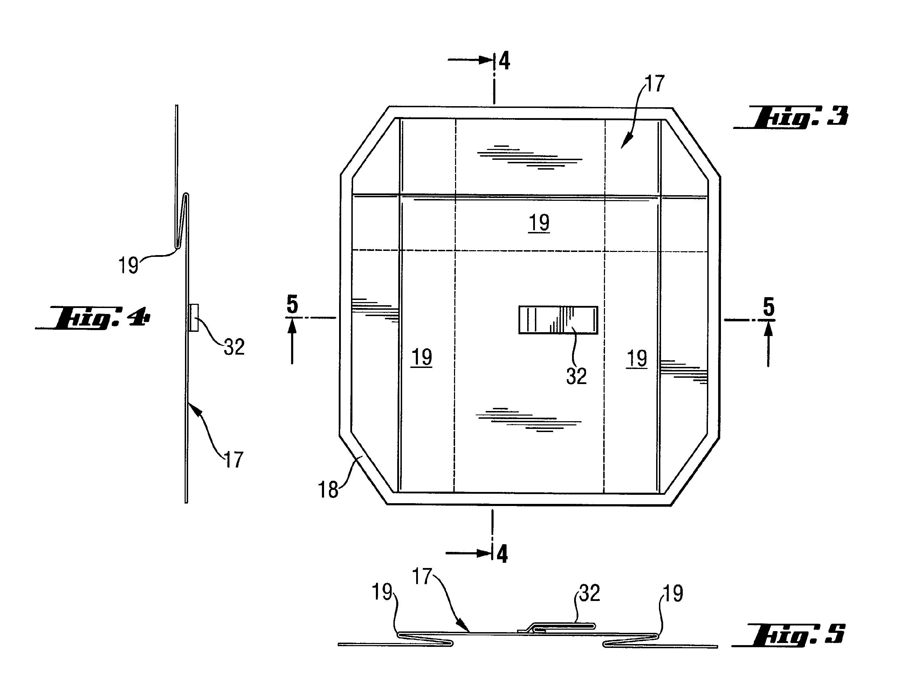

Preferred folding patterns comprise a equal number of folds running in longitudinal direction to either side of the aperture (21), highly preferred are one or two folds on either side, which are preferably disposed symmetrically. A preferred folding pattern will also comprise transversal folds. A highly preferred folding pattern is shown in Figures 3 to 5 and comprises two longitudinal folds and one transversal fold situated above the aperture (21), the traversal folding being carried out after the longitudinal folding.

The term "longitudinal" as used with regard to the human waste collection bag is to be understood as follows: the plane which bisects an upright standing wearer in a left and a right half shall comprise the longitudinal direction of the human waste collection bag. The longitudinal axis is typically an axis of symmetry of the pouch (11).

To facilitate the unfolding of the pouch (11), the garment facing portion (17) thereof is provided with an unfolding means (32), such as a string, tab or lobe.

In another important aspect of the present invention the garment facing portion (17) should comprise a malleable material. The material used for the garment facing portion (17) should have a stiffness of less than 20 mm according to the test described hereinafter. Preferably the material should have a stiffness in the range of 1 mm to 18 mm, more preferably 5 mm to 15 mm and most preferably 10 mm to 14 mm.

The term "stiffness" as used herein refers to the Japanese standard test method JIS L1085 5.7; Method A ; 45 degree cantilever. The material is measured in MD and in CD direction, "stiffness" refers to the arithmetic average of the MD and the CD value.

The relatively large size of the garment facing portion (17) and the malleable material used therefore both achieve, alone and synenergistically in combination, largely improved containment properties of the pouch (11). It has been found that some prior art pouches of longish shape get easily entrapped between the legs of a wearer which largely reduces their storage capacity. Further it has been found that pouches with a large wearer facing portion (16) and relatively small garment facing portion (17) easily get entrapped between the buttocks of a wearer in a manner which also reduces their storage capacity.

Moreover it has been found that folds and wrinkles created in a stiff garment facing portion material do not easily unfold even when some pressure is transmitted by faecal material. The form of the pouch and the malleability of the garment facing portion (17) must be selected in view of each other to optimise the containment properties and namely the storage volume accessible in use. Generally a small garment facing portion (17) requires a very malleable material and also folds (19) in the garment facing portion (17) require the use of a malleable material.

In another aspect it has been found that it can be beneficial to have a small wearer facing portion (16), in particular for a faecal collection bag (10) used by a female wearer. When a female wearer suffers from both urinary and faecal incontinence, the wearer may use a faecal collection bag (10) in combination with a pad or diaper. If the wearer facing portion (16) of the faecal collection bag (10) is then chosen too large the faecal collection bag (10) may obstruct the uro-genital area, such that urine wets the wearer facing portion (16) of the faecal collection bag (10) and gives the wearer a moist and clammy feeling while urine is efficiently collected by devices like a diaper or pad.

Therefore the position of the aperture (21) and thereby of the flange (12) should be selected carefully, such that the outer contour of the wearer facing portion (16), which his typically given by the rim (18), does not exceed the outer contour of the flange by more than 80 mm, preferably not by more than 60 mm, yet preferably not by more than 40 mm, most preferably not by more than 30 mm in the perineal area. The distance by which the outer contour of the wearer facing portion (16) exceeds the outer contour of the flange is indicated as "D" in Figure 2.

Pouches (11) according to the present invention can have a variety of shapes. One preferred shape is shown in Figures 1 to 5. In this preferred embodiment the wearer facing portion (16) is of an essentially square shape, the corner areas of the corresponding square, however, being "cut-off', such that an irregular octogon is obtained. Any essentially rectangular shape, much preferably with "cut-off" or flattened corners, is preferred.

When an faecal collection bag (10) for an incontinent adult is provided the pouch (11) as shown in Fig. 2 should measure from 10 cm to 30 cm, preferably 15 cm to 25 cm in the longitudinal direction and from 10 cm to 30 cm, preferably 15 cm to 25 cm in the transversal direction.

The pouch may comprise only a single orifice, the aperture (21), but may also comprise additional orifices or valves. For example, an air and gas outlet means, in the form of an orifice or a valve can be used to allow for the outlet of flatulence gases or the like. Moreover, an air inlet means, eg in the form of a hole in the garment facing portion (17), has proven useful to facilite the quick unfolding of the pouch (11).

The pouch material, and hence the material of the wearer facing portion (16) and the material of the garment facing portion (17), can comprise one or multiple layers, preferably two or three layers. The layer on the inside of the pouch (11), which will typically at least partially come in contact with faecal material or urine is called the inner layer. The outermost layer of the pouch (11), which will typically at least partially come in contact with the skin to the wearer and the garments of the wearer, is called the outer layer.

The layers of the pouch (11) material may comprise any material, preferably so that the pouch (11) is liquid impervious. The layers may in particular comprise any material such as non-wovens or films. In a preferred embodiment of the present invention a laminate may be formed from a non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the pouch (11) material may comprise a non-woven layer. Such material layers present an uneven surface to the skin of the wearer and thus reduce significantly the problem of occlusion and greatly improve skin healthiness.

In one preferred embodiment of the present invention the pouch (11) material comprises two layers. Preferably the outer layer comprises a non-woven layer and the inner layer comprises a film.

In yet another preferred embodiment of the present invention, the pouch (11) material comprises three layers, preferably one film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a film and the other two layers comprise non-wovens.

The non-woven layer or the non-woven layers comprised by the pouch (11) material may be hydrophobic or hydrophilic. If the pouch (11) material does not comprise a film layer, preferably at least one non-woven layer is hydrophobic. As a consequence, fluid penetration is resisted through the wearer facing portion (16) and the garment facing portion (17) of the human waste collection bag. If the pouch (11) material comprises a film or a hydrophobic non-woven layer, further non-woven layers may be hydrophilic.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, cationic, non-ionic and amphoteric surfactants, may be added to further enhance softness and surface smoothness.

Furthermore, the non-woven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

In one embodiment of the present invention the pouch (11) may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the pouch (11) in any suitable manner. For example, the absorbent material may be loosely arranged within the pouch (11) or may be secured to the inner surface of the pouch (11). Any known techniques for securing absorbent material to nonwoven and film substrates may be used to secure the absorbent material to the inner surface of the pouch (11). The absorbent material may also be arranged to have any desired shape or configuration (e.g., rectangular, oval, circular, etc.).

The pouch (11) is provided with an aperture (21) whereby faecal matter or urine is received from the body prior to storage within the pouch (11) cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction.

The flange (12) is attached to the pouch (11) according to any means known to the man skilled in the art which may provide permanent or releasable attachment. Preferably however, the flange is attached to the pouch (11) by adhesive. Typically, the pouch (11) will be attached to the flange, towards the outer periphery of flange so as not to cause any obstruction for the entering faecal matter or urine.

The flange may be provided in any size depending on the wearer group for which the device is intended. Similarly the flange may be provided in any shape and preferably has a symmetrical shape preferably comprising a plurality of lobes (14).

The flange comprises a garment facing portion and a wearer facing portion. In an preferred embodiment these are two large, substantially flat surfaces, however, the flange (12) may also comprise projections, a front projection and/or a rear projection, in case of a faecal collection bag (10) designed to fit the perineal and/or coccygeal area of the wearer and in case of a urine collection bag designed to fit the genital and/or perineal area.

The flange (12) should be made of soft, flexible and malleable material to allow easy placement of the flange (12) to the perianal or uro-genital area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/l, more preferably 50 g/l. Other thermoplastic foam materials, or other suitable plastics sheet materials having the described properties of such foams (i.e., softness, pliability, stretchability, and contractability) might also be used. Preferably, the material of garment facing portion of the flange (12) may extend into the defined aperture area so as to form a skirt or flap of material which prevents unintentional adhesion of the surface edges of the flange (12) defining the aperture (21) to one another during use.

The human waste collection bag further comprises an attachment means to secure the device to the wearer. Such means include straps and more preferably comprises a body-compatible pressure sensitive adhesive applied to the wearer facing portion of the flange (12).

The adhesive is preferably covered with a release means (not shown) in order to protect the adhesive, such as siliconised paper. The adhesive can cover the entire wearer facing portion of the flange (12), more preferably the flange (12) has at least one, preferably two to six non-adhesive portions. These portions may be adhesive free or may contain inactivated or covered adhesives. As is evident from Figure 1, the adhesive is in one preferred embodiment not applied to the entire wearer facing portion of the flange (12), so as to provide lobes (14) on either side of the flange (12) which are non-adhesive and can thereby serve to facilitate placement and removal of the device whilst avoiding contact with the adhesive. These lobes (14) are however preferably also covered by the release means. Before application of the human waste collection bag to the skin of the wearer, the release means if present is removed.

Any medically approved water resistant pressure sensitive adhesive may be used to attach the device to the perianal or uro-genital area of the wearer, such as hydrocolloid adhesives and hydrogel adhesives. Particularly effective adhesives in providing the desired adhesive properties to secure the flange to the skin of the wearer at the sensitive perianal area, whilst allowing for relatively painless application and removal, are formed from crosslinking polymers with a plastisicer to form a 3-dimensional matrix.

The adhesive can be applied to the wearer facing portion of the flange (12) by any means known in the art such as slot coating, spiral, or bead application or printing. Typically the adhesive is applied at a basis weight of from 20g/m² to 2500g/m², more preferably from 500g/m² to 2000g/m² most preferably from 700g/m² to 1500g/m² depending on the end use envisioned. For example, for human waste collection bags to be used for babies the amount of adhesive may be less than for human waste collection bags designed for active adult incontinence sufferers.

In a preferred embodiment the human waste collection bag is provided in a particular configuration prior to use and, preferably, provided together with an applicator.

In one such configuration, the flange (12) is folded along the longitudinal axis to allow easier placement of the flange (12) in-between the buttocks of a wearer and the pouch (11) is preferably folded, which provides numerous advantages. For example, folding of the pouch (11) allows a smaller packaging format of the human waste collection bag, thus reducing the costs for transport and packaging material. Furthermore, the handling of the human waste collection bag is more convenient if the pouch (11) is folded, since the pouch (11) may otherwise cover parts of the applicator e.g. the handling portion. The pouch (11), when not folded, may also cover parts of the body of the wearer, so that the person placing the human waste collection bag cannot sufficiently visually control the placing.

In another preferred prior to use configuration the pouch (11) is provided unfolded. This configuration is particularly suitable for relatively small pouches (11), which can be provided according to the present invention. A relatively small outer contour of the pouch (11) is best achieved, when folds (19) are provided in the garment facing portion (17). Being able to provide the pouch (11) in an unfolded condition allows easier manufacturing and packaging.

Most preferably the applicator and the human waste collection bag in combination are provided in a particular configuration prior to use. This allows ready application of the human waste collection bag with only a few handling steps, since there is no need to have separate items at hand (the applicator and the human waste collection bag) and to position the applicator on the appropriate areas of the human waste collection bag for application.

The applicator may be provided with a means to hold the applicator and the human waste collection bag together. This means will typically also ensure the correct positioning of the applicator relative to the human waste collection bag and help to maintain the folded configuration of the pouch (11), if the bag is folded. Such a means preferably are provided in the form of any string or band, which may be provided in form of a closed loop, such as a rubber band. Such a means may also be provided in form of a clamp or a clip, made from any suitable material such as plastic or metal.

## Claims

1. A human waste collection bag comprising a pouch (11), said pouch (11) comprising a wearer facing portion (16) and a garment facing portion (17), both having a surface area, **characterised in that** said surface area of said garment facing portion (17) is greater than said surface area of said wearer facing portion (16).

2. A human waste collection bag according to claim 1, wherein said surface area of said garment facing portion (17) is at least 10 % greater than said surface area of said wearer facing portion (16).

3. A human waste collection bag according to claim 1 or 2, wherein said garment facing portion (17) is provided with folds (19).

4. A human waste collection bag according to claim 1, 2 or 3, wherein said garment facing portion (17) is provided with two folds (19) oriented in the longitudinal direction.

5. A human waste collection bag according to any one of the preceding claims wherein said garment facing portion (17) and said wearer facing portion (16) each comprise different materials.

6. A human waste collection bag according to any one of the preceding claims wherein said garment facing portion (17) comprises a malleable material.

7. A human waste collection bag according claim 6 wherein said malleable material has a stiffness of less than 20 mm according to the test described herein.

8. A faecal collection bag according to any one of the preceding claims.

9. A faecal collection bag according to claim 8, comprising a flange (12), wherein the outer contour of the wearer facing portion (16) in the perineal area exceeds the outer contour of the flange by no more than 40 mm.

10. A human waste collection bag comprising a pouch (11) **characterised in that** said pouch (11) comprises a material having a stiffness of less than 20 mm according to the test described herein.
